(19) Europäisches Patentamt
European Patent Office
Office européen des brevets

(11) Publication number : **0 542 549 A1**

(12) # EUROPEAN PATENT APPLICATION

(21) Application number : **92310347.7**

(51) Int. Cl.$^5$ : **A61K 7/48**

(22) Date of filing : **12.11.92**

Amended claims in accordance with Rule 86 (2) EPC.

A request for a correction in the description has been filed pursuant to Rule 88 EPC. A decision on the request will be taken during the proceedings before the Examining Division (Guidelines for Examination in the EPO, A-V, 2.2).

(30) Priority : **15.11.91 GB 9124360**

(43) Date of publication of application : **19.05.93 Bulletin 93/20**

(84) Designated Contracting States : **AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Rawlings, Anthony Vincent**
**Unilever Research U.S Inc, 45 River Road**
**Edgewater, NJ 07020 (US)**

(74) Representative : **Tonge, Robert James et al**
**UNILEVER PLC Patent Division Colworth House Sharnbrook**
**Bedford MK44 1LQ (GB)**

(54) **Use of cosmetic composition.**

(57) The use of at least 0.001% by weight of a physiologically active amide derivative as a stratum corneum flexibility enhancer when applied topically within a cosmetic composition. Increased stratum corneum flexibility treats/prevents cracking and flaking of the kin and additionally smooths fine lines and wrinkles.

EP 0 542 549 A1

## FIELD OF THE INVENTION

The invention relates to the use of physiologically active amide derivatives in improving the flexibility of human skin when applied topically.

## BACKGROUND TO THE INVENTION AND PRIOR ART

It is generally understood that ceramides present within the intercellular lipid lamellae of the stratum corneum play an important rôle in the production and maintenance of the water permeability barrier of the skin. Their importance in stratum corneum flexibility is, however, poorly understood.

Ceramides, or substances closely related to them, have been disclosed as components of skin care compositions for maintaining stratum corneum water barrier function. In particular, Kao Corporation in EP 0 227 994, EP 0 282 816 and EP 0 398 272 discloses synthetic analogues of ceramides which, to a significant extent, have properties similar to natural ceramides, but are relatively cheaper to produce.

In JP-A-63-192703, Kao Corporation discloses a skin composition which contains extracted naturally occurring skin ceramides including either phytosphingosines or α-hydroxy fatty acid-containing ceramides. A further family of ceramides of the type found in skin is disclosed in EP 0 097 050 (Unilever). This highlights the vital rôle played by ω-(O-linoleoyl) ceramides in the water barrier of the skin.

We have now discovered that ceramides and other physiologically active amide derivatives when applied topically to human skin render the lipid matrix of the stratum corneum more extensible, which activity is recognised by the consumer as an improvement in skin flexibility.

The improvements in skin flexibility are particularly important in low humidity conditions, eg during the winter months, when skin dryness can occur. If skin flexibility is not maintained under these conditions, cracking and flaking of the skin can develop. Skin flexibility is also reduced during skin ageing and an increase in skin flexibility smooths fine lines and wrinkles.

Our investigations have now shown that these amide derivatives are capable of improving the extensibility of stratum corneum under low humidity conditions and can thereby maintain skin flexibility. This recently discovered property of the ceramides and other physiologically active amide derivatives is accordingly quite distinct from the previously reported properties of some of these materials in maintaining or otherwise influencing the water permeability barrier of the skin.

## SUMMARY OF THE INVENTION

The invention accordingly provides for the use of at least 0.001% by weight based on the total composition of a physiologically active amide derivative as a stratum corneum flexibility enhancer in a cosmetic composition for topical application to skin, said composition otherwise comprising a major proportion of a cosmetically acceptable vehicle for the amide derivative.

## DISCLOSURE OF THE INVENTION

### The physiologically active amide derivative

The physiologically active amide derivative is characterised by an unsaturated N-acylated fatty acid group, the unsaturation occurring in either the N-acyl moiety or the fatty acid moiety, or in both moieties.

By "physiologically active" we mean that the amide derivative is capable of increasing the extensibility of the lipid matrix of the stratum corneum.

The physiologically active amide derivative preferably has the structure (1):

$$Y-(O)_n-C_aH_b-\overset{\overset{O}{\|}}{C}-\overset{\overset{A}{|}}{N}-\overset{\overset{B}{|}}{CH}-D \qquad (1)$$

where

A is -H or $-CH_2CH_2OH$

B is

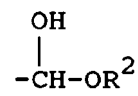

or

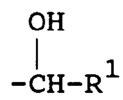

and

$R^1$ is $C_{8-28}$ alkyl,

$R^2$ is a $C_{8-28}$ alkyl or the group $-CH(OH)R^2$

D is -H or $-CH_2OH$

Y is -H or a residue of an all cis n-6,9 fatty acid or a derivative thereof, having the structure (2)

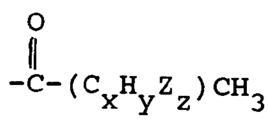

(2)

where

Z is -OH or an epoxy oxygen

x is an integer of from 16 to 20

y is an integer of from 24 to 36, and

z is O, or an integer of from 1 to 4;

and where

a is an integer of from 7 to 49

b is an integer of from 10 to 98

n is 0 or 1

provided also that any -OH group can be phosphorylated, sulphated or glycolated, the phosphorylated group, when present, being represented by one of the following:

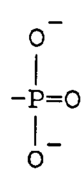

or

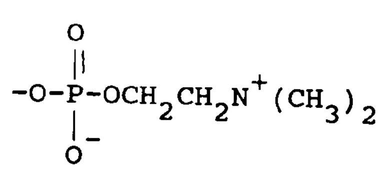

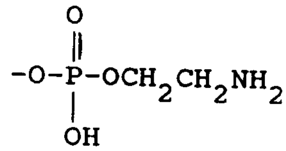

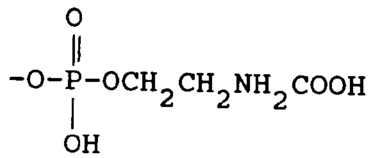

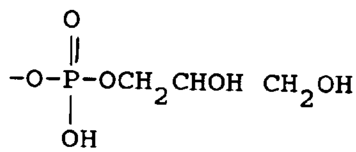

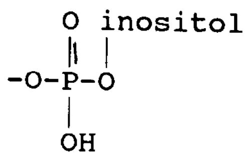

A preferred subclass of amide derivatives having the structure (1) are naturally occurring ceramides and sphingosines where, in the structure (1),

A is -H

B is

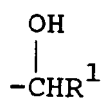

D is $-CH_2OH$

A particularly preferred example of a naturally occurring sphingosine is:

ω-(O-linoleoyl) sphingosine having the structure (10):

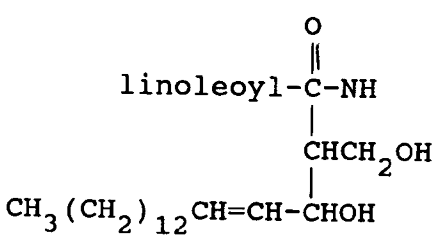

(10)

A particularly preferred subclass of naturally occurring ceramides is:

ω-(O-linoleoyl) ceramides having the structure (11):

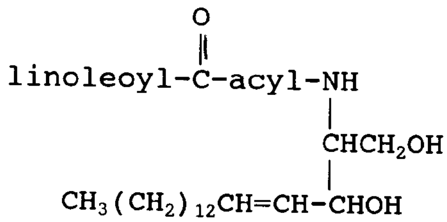

(11)

Specific examples of which are those where the acyl group is saturated and have the structures (12) or (13):

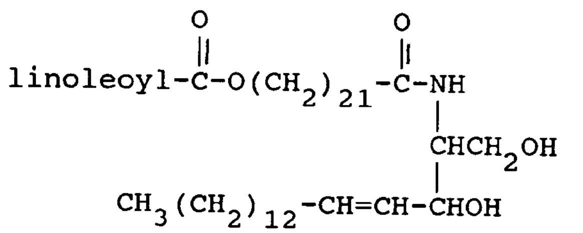

(12)

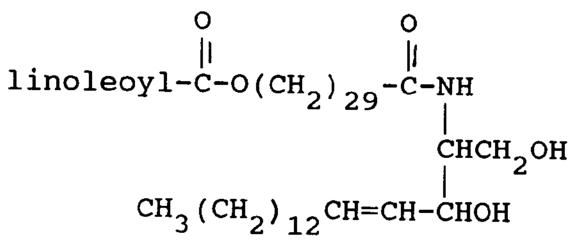

(13)

or those where the acyl group is unsaturated and, one of which has the structure (14):

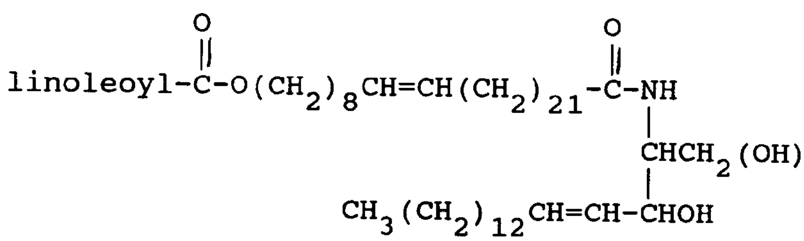

(14)

A further preferred subclass of the amide derivatives having the structure (1) are synthetic derivatives, such as pseudosphingosines and pseudoceramides where, in structure (1),

A is $-CH_2CH_2OH$

B is

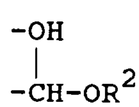

or

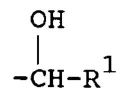

and D is -H.

A particularly preferred sub-class of pseudoceramides for use in accordance with the invention includes ω-(O-linoleoyl) ceramides having the structure (15):

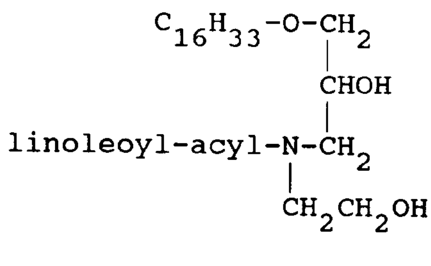

(15)

Specific examples of which are those where the acyl group is saturated, such as that having the structure (16):

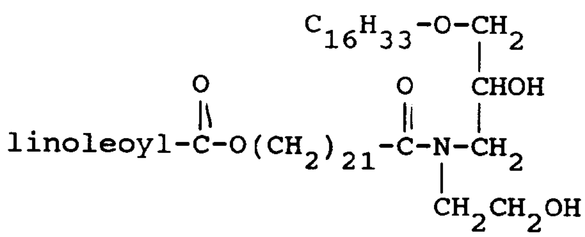

(16)

or where the acyl group is unsaturated, such as that having the structure (17):

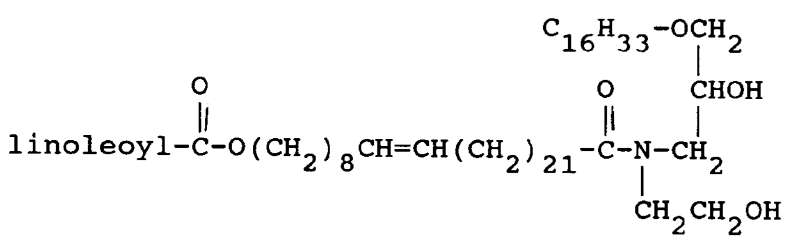

(17)

Further particularly preferred examples of pseudosphingosines are:

N-(2-hydroxy-3-hexadecyl oxypropyl)-N- (2-hydroxyethyl)-linoleoylamide having the structure (18):

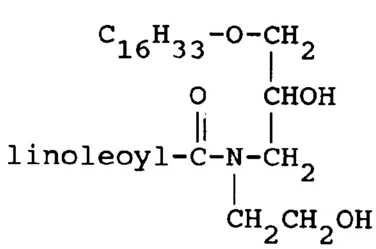

(18)

N-(2,3-dihydroxyoctadecyl)-N-(2-hydroxyethyl)-oleoylamide having the structure (19):

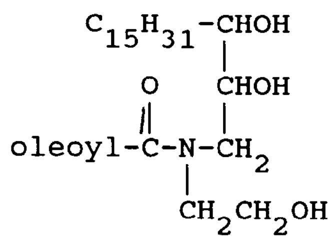

(19)

N-(2-hydroxyoctadecyl)-N-(2-hydroxyethyl)-2-linoleoylamine having the structure (20):

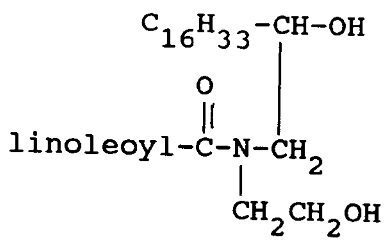

(20)

N-(2-hydroxyoctadecyl)-N-(2-phosphoethyl)-2-oleoylamide having the structure (21):

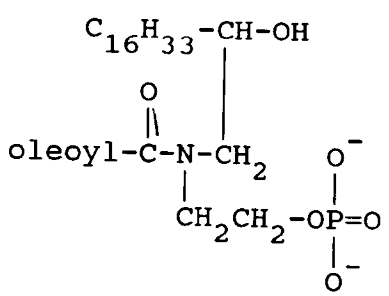

(21)

Arachidonoyl diphosphosphingosine, having the structure (22):

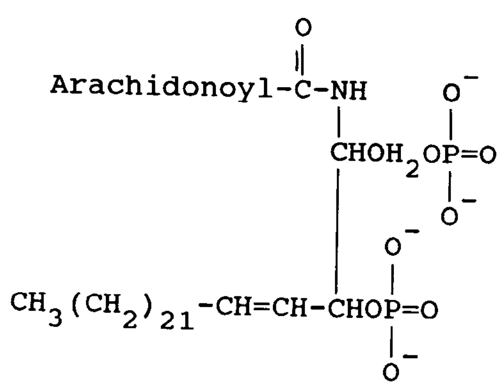

(22)

It is to be understood that the foregoing structures are exemplary of many physiologically active amide derivatives that can be used in performance of the invention.

It is further understood that the amide derivatives can be used individually or as mixtures.

The amount of the physiologically active amide deriviative present in the composition for use in accordance with the invention is from 0.001 to 10%, preferably 0.1 to 1% by weight.

The cosmetically acceptable vehicle

The composition for use according to the invention also comprises a cosmetically acceptable vehicle to

act as a dilutant, dispersant or carrier for the amide derivative in the composition, so as to facilitate its distribution when the composition is applied to the skin and/or hair.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate. The cosmetically acceptable vehicle will usually form from 10 to 99.9%, preferably from 50 to 99% by weight of the emulsion, and can, in the absence of other cosmetic adjuncts, form the balance of the composition.

Optional skin benefit materials and cosmetic adjuncts

A particularly convenient form of the composition according to the invention is an emulsion, in which case an oil or oily material will normally be present, together with an emulsifier to provide either a water-in-oil emulsion or an oil-in-water emulsion, depending largely on the average hydrophilic-lypophilic balance (HLB) of the emulsifier employed.

Oil or oily material

The composition according to the invention can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

The amount of emulsifier or mixtures thereof, to be incorporated in the composition of the invention, when appropriate is from 1 to 50%, preferably from 2 to 20% and most preferably from 2 to 10% by weight of the composition.

Water

The composition of the invention can also comprise water, usually up to 98%, preferably from 5 to 80% by volume.

Silicone Surfactant

The composition of the invention can also optionally comprise a high molecular weight silicone surfactant which can also act as an emulsifier in place of or in addition to other emulsifying agents.

The silicone surfactant is usually a high molecular weight polymer of dimethyl polysiloxane with polyoxyethylene and/or polyoxypropylene side chains having a molecular weight of from 10,000 to 50,000.

The dimethyl polysiloxane polymer when present is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

Other Cosmetic Adjuncts

Examples of conventional adjuncts which can optionally be employed include preservatives, such as parahydroxy benzoate esters; antioxidants, such butyl hydroxy toluene; humectants, such as glycerol, sorbitol, 2-pyrrolidone-5-carboxylate, dibutylphthalate, gelatin, polyethylene, glycol, preferably PEG 200-600; buffers, such as lactic acid together with a base such as triethanolamine or sodium hydroxide; surfactants, such as glycerol ethers and other ceramides of synthetic, animal or plant origin; phospholipids; waxes, such as beeswax, ozokerite wax, paraffin wax, plant extracts, such as Aloe vera, cornflower, witch hazel, elderflower, cucumber; thickeners; activity enhancers; colourants; perfumes; and sunscreen materials such as ultrafine titanium dioxide and organic sunscreens such as p-aminobenzoic acid and esters thereof, ethylhexyl p-methoxycinnamate, 2-ethoxyethyl p-methoxycinnamate and butyl methoxydibenzoylmethane, and mixtures thereof.

In a further preferred composition, the amide derivative, or a mixture thereof, is combined with cholesterol, cholesterol fatty acids, fatty acids, triglycerides, cerebroside, phospholipid and other ingredients well known to those skilled in the art to produce a liposomal dispersion.

In yet another preferred composition, the amide derivative, or a mixture thereof, is dissolved in squalene or squalane and formulated with volatile and non-volatile silicones to produce an anhydrous or nearly anhydrous single phase system.

Cosmetic adjuncts can form the balance of the composition.

Use of the Composition

The composition according to the invention is intended primarily for topical application to human skin for plasticising the lipid matrix of the stratum corneum so as to render it more extensible. The flexibility of the skin is thereby enhanced. Such enhancement of flexibility is important particularly for treatment of skin dryness and skin ageing effects such as fine lines.

In use, a small quantity of the compositions, for example from 1 to 5g, is applied to areas of the skin that require treatment, and rubbed in.

METHOD OF TREATMENT

The invention also provides a method for the treatment of the stratum corneum to enhance flexibility which method of treatment comprises topically applying to the skin at least 0.001% by weight based on the total composition of a physiologically active amide derivative, said composition otherwise comprising a major proportion of a cosmetically acceptable vehicle for the amide derivative.

PRODUCT FORM AND PACKAGING

The composition of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity

and intended use by the consumer.

For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

The invention accordingly also provides a closed container containing a cosmetically acceptable composition as herein defined.

In vitro measurement of the stratum corneum extensibility

The benefits of the composition following topical application to skin can be quantified by in vitro measurements employing pig skin and an extensometer, in accordance with the following procedure.

1. Preparation of Stratum Corneum from Pig Epidermis

Pig skin was selected for this evaluation and this was first cleaned with an aqueous ethanol-chlorohexidine solution (15 : 75 : 10:, Hibitane; ICI Pharmaceuticals). Hairs were removed by clipping followed by depilation with commercially-available thioglycolate cream (Immac : Whitehall Laboratories) and 0.4mm thick slices of skin were removed using a Davies electric skin dermatome. Stratum corneum was isolated by incubating strips of skin, dermis side down, into a solution of trypsin (0.25g% Flow Laboratories) and phosphate-buffered saline (PBS) comprising 137mM NaCl; 2.68mM KCl; 8.1mM $NaHPO_4$; 1.47mM $KH_2PO_4$ at pH 7.4 for 2-4h at 37°C. The dermis was separated from the epidermis using forceps and the latter was incubated in fresh trypsin-PBS solution overnight at 4°C. The enzyme solution was changed several times on the following day before the stratum corneum was washed with sonication (Lucas Dawe Ultrasonics; bath sonicleaner) and three changes of PBS to remove trypsin and residual cells. The corneum was then washed with distilled water containing 0.02% sodium azide (to inhibit bacterial growth) before floating onto fluorocarbon filter (Spectra-mesh; 149μ mesh). After drying at ambient temperature and humidity the sheets of corneum were cut into rectangular strips measuring 1 x 2.5cm. The pieces of corneum were mounted at each end with plastic tape (Flow tape; Flow Laboratories) and a 6mm hole was punched into the centre of each tab. During this whole procedure the orientation of the stratum corneum was maintained to ensure samples were treated on their external surfaces only in subsequent experiments. Samples were stored in petri-dishes at ambient humidity and temperature until required.

2. Sample treatment

Corneum samples were pre-equilibrated to 44% relative humidity by suspending from hooks in humidity chambers containing a saturated solution of magnesium nitrate.

To examine the influence of compositions according to the invention or control samples on corneum extensibility, a sample (10μl or 50μl) was applied to the external surface of the corneum and rubbed in with twenty strokes of a gloved finger.

3. In vitro extensibility

Samples were extended to 2% of their original length at 20mm/min using a linear extensometer (Rheometer; Diastron Ltd) before and after application of the samples and equilibration to 44% relative humidity. A force/extension graph is computed and measurements are reported as an extensibility ratio of their slopes of before and after treatment. Thus, ratios greater than one indicate increases in corenum extensibility.

In addition to the corneum humidity chamber control, all equipment was housed in a humidity and temperature controlled room.

4. Statistical analysis

Results are reported as mean ± standard deviation. Mean values were compared using a students t test. Significance was set at the 5% level.

5. Design of Experiment

The extensibility of pig skin stratum corneum was measured following treatment with ω-(O-linoleoyl)ceramide having the structure (12), as herein defined.

By way of comparison, a sample containing the same ceramide following hydrolysis to cleave the linoleoyl moiety and a further sample containing only linoleoate were also applied to stratum corneum and extensibility measurements performed in the same manner.

The level of application of each sample was 4µl/cm² (25ng) in each case.

6. Results

The extensibility ratios were as follows:

| Sample | Extensibility ratio |
|---|---|
| Ceramide structure (12) | 2.11 ± 0.45 |
| Hydrolysed ceramide structure (12) | 1.44 ± 0.20 |
| Linoleate | 1.29 ± 0.16 |

7. Conclusions

From the above results, it can be concluded that the extensibility ratio for the stratum corneum sample treated with ceramide structure (12) was significantly greater than that treated with either hydrolysed ceramide structure (12) [consisting essentially of linoleate and a saturated acyl sphingosine] or linoleate itself. This underlines the importance of the intact physiologically active amide derivative possessing an unsaturated N-acyl moiety or an unsaturated fatty acid moiety, this activity, ie the ability to induce superior stratum corneum extensibility, being severely reduced with the cleavage of the unsaturated fatty acid moiety from the amide derivative.

EXAMPLES

The invention is illustrated by the following examples.

Example 1

This example illustrates a high internal phase water-in-oil emulsion in accordance with the invention.

A high internal phase water-in-oil emulsion having the following formulation was prepared:

| | % w/w |
|---|---|
| Fully hydrogenated coconut oil | 3.9 |
| Sphingosine having the structure (10) | 0.1 |
| Brij 92* | 5 |
| Bentone 38 | 0.5 |
| Preservative | 0.3 |
| $MgSO_4 7H_2O$ | 0.3 |
| Butylated hydroxy toluene | 0.01 |
| Perfume | qs |
| Water | to 100 |

*Brij 92 is polyoxyethylene (2) oleyl ether

Example 2

This example also illustrates a high internal phase water-in-oil emulsion in accordance with the invention in which the formulation of Example 1 was prepared but with the following changes:

i. liquid paraffin replaced the fully hydrogenated coconut oil, and

ii. ceramide having the structure (12) was used in place of the sphingosine having the structure (10).

Example 3

This example also illustrates a high internal phase water-in-oil emulsion in accordance with the invention in which the formulation of Example 1 was prepared but with the following changes:

Ceramide having the structure (13) was used in place of the sphingosine having the structure (10).

Example 4

This example illustrates an oil-in-water cream containing an ester of the invention.

An oil-in-water cream emulsion having the following formulation was prepared:

| | % w/w |
|---|---|
| Mineral oil | 4 |
| Ceramide having the structure (14) | 0.1 |
| Brij 56* | 4 |
| Alfol 16RD* | 4 |
| Triethanolamine | 0.75 |
| Butane-1,3-diol | 3 |
| Xanthan gum | 0.3 |
| Preservative | 0.4 |
| Perfume | qs |
| Butylated hydroxy toluene | 0.01 |
| Water | to 100 |

*Brij 56 is cetyl alcohol POE (10)

Alfol 16RD is cetyl alcohol

Example 5

This example also illustrates an oil-in-water emulsion containing an ester of the invention, in which the formulation of example 4 was prepared but with the following change:

the pseudoceramide having structure (16), as herein defined, was used in place of the ceramide having the structure (14).

Example 6

This example also illustrates an oil-in-water emulsion in accordance with the invention, in which the formulation of example 4 was prepared but with the following changes:

the pseudoceramide having the structure (17) as herein defined, was used in place of the ceramide having the structure (14).

Example 7

This example illustrates an alcoholic lotion containing an amide of the invention.

The lotion had the following formulation:

| | % w/w |
|---|---|
| Pseudosphingosine having the structure (18) | 0.2 |
| Ethanol | 40 |
| Perfume | qs |
| Butylated hydroxy toluene | 0.01 |
| Water | to 100 |

Example 8

This example illustrates an alcoholic lotion containing an amide of the invention.

The lotion had the following formulations:

| | % w/w |
|---|---|
| Pseudosphingosine having the structure (19) | 0.2 |
| Dimethylsulphoxide | 10 |
| Ethanol | 40 |
| Antioxidant | 0.1 |
| Perfume | qs |
| Water | to 100 |

Examples 9 and 10

The following compositions according to the invention represent lotions which can be used in the treatment of dry skin:

| | % w/w | |
|---|---|---|
| | 9 | 10 |
| Pseudosphingosine having the structure (20) | 1.5 | - |
| Pseudosphingosine having the structure (21) | - | 0.5 |
| Perfume | 0.1 | 0.1 |
| Hydroxyethyl cellulose | 0.4 | 0.4 |
| Absolute ethanol | 25 | 25 |
| p-methyl benzoate | 0.2 | 0.2 |
| Sterilised demineralised water | to 100 | to 100 |

Examples 11 and 12

The following compositions according to the invention represent lotions which can be used in the treatment of dry skin:

| | % w/w | |
|---|---|---|
| | 11 | 12 |
| The pseudosphingosine having the structure (22) | 0.08 | - |
| The pseudosphingosine having the structure (10) | - | 0.15 |
| Ethanol | 10 | 10 |
| Perfume | 0.5 | 0.5 |
| Distilled water | to 100 | to 100 |

Example 13

This example illustrates a high internal phase water-in-oil emulsion in accordance with the invention. A high internal phase water-in-oil emulsion having the following formulation was prepared:

| | % w/w |
|---|---|
| Fully hydrogenated coconut oil | 3.9 |
| Ceramide having the structure (12) | 0.1 |
| Brij 92* | 5 |
| Bentone 38 | 0.5 |
| Preservative | 0.3 |
| $MgSO_4 7H_2O$ | 0.3 |
| Butylated hydroxy toluene | 0.01 |
| Perfume | qs |
| Water | to 100 |

*Brij 92 is polyoxyethylene (2) oleyl ether

**Claims**

1. The use of at least 0.001% by weight based on the total composition of a physiologically active amide derivative as a stratum corneum flexibility enhancer in a cosmetic composition for topical application to skin, said composition otherwise comprising a major proportion of a cosmetically acceptable vehicle for the amide derivative.

2. The use of a stratum corneum flexibility enhancer in accordance with claim 1, in which the physiologically active amide derivative has the structure (1), as herein defined.

3. The use of a stratum corneum flexibility enhancer in accordance with claim 1 wherein the physiologically active amide derivative is a ceramide or pseudoceramide.

4. The use of a stratum corneum flexibility enhancer in accordance with claims 1 or 2, in which the physiologically active amide derivative is a naturally occurring sphingosine derivative, where, in the structure (1),

A is -H
B is

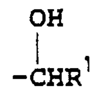

D is $-CH_2OH$
m is 0
n is 0
Y has the structure (2); and
$R^1$ is a $C_{8-28}$ alkenyl

5. The use of a stratum corneum flexibility enhancer in accordance with claim 4, in which the naturally occurring sphingosine derivative has the structure (10), as herein defined.

6. The use of a stratum corneum flexibility enhancer in accordance with claims 1 or 2, in which the physiologically active amide is a naturally occurring ceramide where in structure (1)

A is H
B is

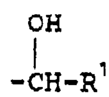

D is $CH_2OH$
m is 1
n is 1
Y has the structure (2); and
$R^1$ is a $C_{8-28}$ alkenyl

7. The use of a stratum corneum flexibility enhancer in accordance with claim 6, in which the naturally occurring ceramide has the structure (11), as herein defined.

8. The use of a stratum corneum flexibility enhancer in accordance with claim 7, in which the physiologically active amide derivative is a naturally occuring ceramide chosen from those having the structures (12), (13) and (14) as herein defined.

9. The use of a stratum corneum flexibility enhancer in accordance with claim 1 or 2, in which the physiologically active amide derivative is a pseudoceramide where, in structure (1),

A is $-CH_2CH_2OH$
B is

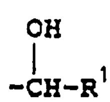

D is -H
m is 1
n is 1
Y has the structure (2); and
$R_1$ is the group $-CH_2-O-R$

**10.** The use of a stratum corneum flexibility enhancer in accordance with claim 9, in which the pseudoceramide has the structure (15), as herein defined.

**11.** The use of a stratum corneum flexibility enhancer in accordance with claim 10, in which the pseudoceramide is chosen from those having the structures (16) and (17) as herein defined.

**12.** The use of stratum corneum flexibility enhancer in accordance with claims 1 or 2, in which the physiologically active amide is a pseudoceramide derivative where in structure (1)

A is $CH_2CH_2OH$
B is

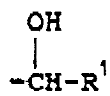

D is $CH_2OH$
m is 0
n is 0; and
Y has the structure (2)

**13.** The use of a stratum corneum flexibility enhancer in accordance with claim 12, in which the pseudosphingosine is chosen from those having the structure (18), (19), (20) and (21), as herein defined.

**14.** The use of stratum corneum flexibility enhancer in accordance with any preceding claim, in which the amount of the physiologically active amide derivative in the composition is from 0.001 to 10% by weight.

**15.** The use of a stratum corneum flexibility enhancer in accordance with any preceding claim for topical application to skin to remove fine lines and wrinkles.

European Patent Office

# PARTIAL EUROPEAN SEARCH REPORT

which under Rule 45 of the European Patent Convention shall be considered, for the purposes of subsequent proceedings, as the European search report

Application Number
EP 92 31 0347
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | PATENT ABSTRACTS OF JAPAN vol. 11, no. 133 (C-418)(2580) & JP-A-61 271 205 ( KANEBO ) * abstract * --- | 1-2, 11-12 | A61K7/48 |
| X | EP-A-0 455 429 (UNILEVER) * the whole document * --- | 1-12 | |
| X,P | EP-A-0 495 624 (UNILEVER) * page 15, lines 55-58 * * page 1, line 1 - page 9, line 12; claims 1-6; examples 1-24 * --- | 1-12 | |
| X,P | EP-A-0 482 860 (UNILEVER) * the whole document * --- | 1-12 | |
| X | WO-A-9 001 323 (BERNSTEIN) * page 3, lines 22-24, page 4, lines 13-16, * * claims 1-26; examples 1-2 * --- -/-- | 1-2, 11-12 | TECHNICAL FIELDS SEARCHED (Int. Cl. 5): A61K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 19 JANUARY 1993 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 92 31 0347
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
| --- | --- | --- | --- |
| X | EP-A-0 420 722 (L'OREAL)<br>* the whole document *<br>--- | 1-12 | |
| P,X | US-A-5 118 507 (CLEMENT)<br>* column 3, lines 1-3 *<br>--- | 1-2, 11-12 | |
| P,X | WO-A-9 206 982 (UNILEVER)<br>* the whole document *<br>----- | 1-12 | |

TECHNICAL FIELDS SEARCHED (Int. Cl. 5)

EPO FORM 1503 03.82 (P04E10)

EP 92 31 0347 -C-

Claims searched completely : 1,2,7-12
Claims searched incompletely : 3-6

Reason : The term sphingosine used in claims 3-6 described an amine and not an amide. Attachment of a fatty acid to the amino group of sphingosine gives rise to a ceramide. Sphingose was excluded from the search.